(19) Europäisches Patentamt
European Patent Office
Office européen des brevets

(11) Veröffentlichungsnummer : **0 463 988 A2**

# (12) EUROPÄISCHE PATENTANMELDUNG

(21) Anmeldenummer : **91810437.3**

(51) Int. Cl.[5] : **B01D 61/24,** C07C 51/42

(22) Anmeldetag : **11.06.91**

(30) Priorität : **19.06.90 CH 2037/90**

(43) Veröffentlichungstag der Anmeldung :
**02.01.92 Patentblatt 92/01**

(84) Benannte Vertragsstaaten :
**CH DE FR GB IT LI**

(71) Anmelder : **CIBA-GEIGY AG**
**Klybeckstrasse 141**
**CH-4002 Basel (CH)**

(72) Erfinder : **Berger, Joseph, Dr.**
**Sperrstrasse 40/18**
**CH-4057 Basel (CH)**
Erfinder : **Feldkamp, Thomas**
**Thermenstrasse 11**
**CH-4310 Rheinfelden (CH)**
Erfinder : **Lohse, Friedrich, Prof. Dr.**
**Buchenstrasse 23**
**CH-4104 Oberwil (CH)**
Erfinder : **Müller, Manfred, Dr.**
**Am Kreuzberg 13**
**CH-6252 Dagmersellen (CH)**

(54) **Verfahren zur Anreicherung oder Trennung von organischen Stoffgemischen.**

(57) Salze organischer Carbonsäuren können von nicht salzartigen organischen Verbindungen mit Hilfe einer semipermeablen Membran aus einem Perfluorsulfonsäurepolymer oder Salzen dieser Polymeren getrennt werden, indem man eine Lösung der Salze und der organischen Verbindung in einem $C_1$-$C_4$-Alkanol mit einer Seite der Membran in Berührung bringt und sich auf der Gegenseite der Membran das reine Lösungsmittel befindet. Das Verfahren kann z.B. in Reinigungsverfahren oder zur Rückgewinnung von Reaktionskomponenten aus Reaktiosrückständen eingesetzt werden.

EP 0 463 988 A2

Jouve, 18, rue Saint-Denis, 75001 PARIS

Die vorliegende Erfindung betrifft ein Verfahren zur Anreicherung oder Trennung von Salzen organischer Carbonsäuren von nicht salzartigen organischen Verbindungen, bei dem man eine Lösung dieser Salze und Verbindungen in einem niedermolekularen Alkanol mit einer Seite einer semipermeablen Membran aus einem Perfluorsulfonsäurepolymer in Berührung bringt, wobei sich auf der Gegenseite der Membran das reine niedermolekulare Alkanol befindet.

In der US-A-4 846 977 ist ein Trennverfahren für Gemische von polaren und nichtpolaren Flüssigkeiten mit Hilfe von semipermeablen Membranen aus Perfluorsulfonsäurepolymeren beschrieben. Als polare Flüssigkeiten kommen z.B. niedere Alkanole und besonders Wasser und als nichtpolare Flüssigkeiten z.B. Kohlenwasserstoffe, Ether, Ketone, Ester oder organische Säuren in Frage.

Es wurde überraschend gefunden, dass mit den gleichen Membranen auch Mischungen aus Salzen von organischen Carbonsäuren und nicht salzartigen organischen Verbindungen in Form von Lösungen in niedermolekularen Alkanolen angereichert oder getrennt werden können.

Gegenstand der Erfindung ist ein Verfahren zum Anreichem oder Trennen von Salzen organischer Carbonsäuren von nicht salzartigen organischen Verbindungen mit einer semipermeablen Membran aus einem Perfluorsulfonsäurepolymer oder Salzen dieser Polymeren, das dadurch gekennzeichnet ist, dass man eine Lösung der Salze und organischen Verbindungen in einem unsubstituierten oder mit $C_1$-$C_3$-Alkoxy substituierten $C_1$-$C_4$-Alkanol, Mischungen dieser Alkanole oder Mischungen der Alkanole mit Ethern mit einer Seite der Membran in Berührung bringt und sich auf der Gegenseite der Membran das reine Lösungsmittel befindet.

Die Konzentration der Salze und organischen Verbindungen beträgt bevorzugt 0,0001 bis 10, besonders 0,001 bis 5 und insbesondere 0,001 bis 3 Gewichtsprozent, bezogen auf die Lösung.

Die Dicke der Membran kann z.B. 5 bis 300 μm, bevorzugt 20 bis 200 μm betragen.

Perfluorsulfonsäurepolymere und Salze dieser Polymeren sind bekannt und z.B. in der US-A-4,846,947 beschrieben. Einige dieser Polymeren sind unter dem Markennamen NAFION® (DuPont) kommerziell erhältlich.

In einer bevorzugten Ausführungsform besteht die Membran aus einem Perfluorsulfonsäurepolymer mit wiederkehrenden Strukturelementen der Formel I

$$-\left[CF_2\text{-}C(R_1)_2\right]_w-CF_2\text{-}CF\text{-}\left(O\text{-}CF_2\text{-}CFR_2\right)_x\left[O(CFR_2)_y\right]_z-SO_3M \qquad (I)$$

worin die $R_1$ und $R_2$ unabhängig voneinander für F oder $C_1$-$C_{10}$-Perfluoralkyl stehen, w eine Zahl von 5 bis 15 bedeutet, x eine Zahl von 0 bis 6 darstellt, y eine Zahl von 1 bis 16 darstellt, z eine Zahl von 0 bis 16 ist, und M für $H^{\oplus}$, ein Ammoniumkation oder ein Metallkation steht. $R_1$ und $R_2$ sind bevorzugt Fluor oder $C_1$-$C_3$Perfluoralkyl, besonders bevorzugt Fluor oder Trifluormethyl und insbesondere bevorzugt Fluor. In Formel I stehen bevorzugt w für eine Zahl von 5 bis 10, x für eine Zahl von 0 bis 2, y für eine Zahl von 1 bis 6 und z für eine Zahl von 0 bis 6, besonders 0 bis 2.

M kann als Ammoniumkation $NH4^{\oplus}$ oder ein Ammoniumkation eines primären, sekundären oder tertiären offenkettigen Amins mit bevorzugt 1 bis 20, besonders 1 bis 12 C-Atomen bedeuten, oder ein Ammoniumkation eines monocyclischen oder bicyclischen sekundären oder tertiären oder eines tricyclischen tertiären Amins mit bevorzugt 4 bis 12 C-Atomen sein.

M in der Bedeutung eines Metallkations kann ein ein- bis dreiwertiges Kation der Metalle der Haupt- und Nebengruppen, der Uebergangsmetalle und der Edelmetalle sein. Bevorzugt sind ein- oder zweiwertige Metallkationen. Beispiele für Metalle sind Li, Na, K, Rb, Cs, Mg, Ca, Sr, Ba, In, Sn, Pb, Cu, Ag, Au, Zn, Cd, Hg, Cr, Mo, Mn, Fe, Co, Ni, Rn, Rh, Pd, Ir, Pt, Sb, Bi, sowie die Gruppe der Seltene-Erdmetalle. Bevorzugte Metalle sind die Alkali- und Erdalkalimetalle, Cu, Ag, Au, Fe, Co, Ni, Zn, Cd und Mn.

In einer bevorzugten Ausführungsform steht M für $NH4^{\oplus}$, ein Ammoniumkation mit insgesamt 1 bis 18 C-Atomen, oder ein ein- bis dreiwertiges Metallkation. Besonders bevorzugt steht M für ein Alkalimetallkation oder $Ag^{\oplus}$.

Die Permeation eines Salzes einer organischen Carbonsäure bzw. einer nicht salzartigen organischen Verbindung kann durch die Wahl des Kations M beeiflusst werden. Bei Verwendung von Membranen mit kleineren einwertigen Kationen permeiert im allgemeinen bevorzugt die nicht salzartige Verbindung. Bei Verwendung von grösseren ein- oder mehrwertigen Kationen permeiert im allgemeinen bevorzugt das Salz.

Es wurde gefunden, dass in besonders günstigen Fällen das Salz praktisch vollständig zurückgehalten wird, wenn M in Formel I für $Ag^{\oplus}$ steht. Umgekehrt wird die unpolarere organische Verbindung praktisch vollständig zurückgehalten, wenn M in Formel I für $Cs^{\oplus}$ steht. Bevorzugt ist daher ein Verfahren, bei dem in Formel I M für $Ag^{\oplus}$ oder $Cs^{\oplus}$ steht.

Das als Lösungsmittel verwendete Alkanol enthält 1 bis 4 C-Atome und bevorzugt 1 bis 3 C-Atome. Es kann z.B. mit Methoxy oder Ethoxy substituiert sein. Beispiele sind Methanol, Ethanol, n- und i-Propanol, n-, i- und t-Butanol, Methoxyethanol, Ethoxy-ethanol, Propoxyethanol, 1-Methoxy-propan-3-ol, 2-Methoxy-propan-1-ol. Bevorzugte Lösungsmittel sind Methanol, Ethanol, 1- oder 2-Propanol und 2-Methoxyethanol. Es können auch Mischungen der Alkanole untereinander oder mit Ethern, z.B. Diethylether oder Ethylenglykoldimethylether verwendet werden.

Bei dem Salz der organischen Carbonsäure kann es sich um Ammonium- oder Metallsalze handeln, z.B. $NH_4^{\oplus}$, einem Ammoniumkation eines primären, sekundären oder tertiären Amins mit insgesamt 1 bis 20 C-Atomen, Alkalimetallsalze und Erdalkalimetallsalze. Bevorzugt sind Alkalimetallsalze und Ammoniumsalze. Besonders bevorzugt sind $NH4^{\oplus}$-und $Li^{\oplus}$-Salze.

Bei der organischen Carbonsäure kann es sich z.B. um Mono-, Di-, Tri- oder Tetracarbonsäuren handeln. Bevorzugt sind aliphatische, cycloaliphatische, aromatische und heterocyclische oder heteroaromatische Monocarbonsäuren, die 1 bis 18 C-Atome, vorzugsweise 1 bis 12 C-Atome enthalten.

Eine bevorzugte Ausführungsform ist dadurch gekennzeichnet, dass die organische Säure der Formel (II)

$$R_3\text{-X-COOH} \qquad \text{(II)}$$

entspricht, worin X eine direkte Bidung, $C_1$-$C_4$-Alkylen, $C_2$-$C_4$-Alkyliden oder $C_2$-$C_4$-Alkenylen bedeutet, $R_3$ für H, $C_1$-$C_{12}$-Alkyl, $C_2$-$C_{12}$-Alkenyl, $C_2$-$C_{12}$-Alkinyl, $C_3$-$C_{12}$-Cycloalkyl, $C_3$-$C_{12}$-Cycloalkenyl, $C_6$-$C_{16}$-Aryl, $C_3$-$C_{12}$-Heterocycloalkyl, $C_3$-$C_{12}$-Heterocycloalkenyl, $C_6$-$C_{16}$-Heteroaryl steht, die unsubstituiert oder mit -OH, -SH, -CN, $-NO_2$, Halogen, $C_1$-$C_6$-Alkyl, $C_1$-$C_6$-Alkoxy, $C_1$-$C_6$-Alkylthio, $C_1$-$C_6$-Alkyl-Y- mit Y gleich -CO-, -SO-, $-SO_2$-, -CO-O-, -O-CO-, $-CO-NR_4R_5$-, $-NR_4R_5$-CO- substituiert sind, und $R_4$ und $R_5$ unabhängig voneinander H, $C_1$-$C_6$-Alkyl, $C_2$-$C_4$-Hydroxyalkyl oder $R_4$ und $R_5$ zusammen Tetramethylen, Pentamethylen oder 3-Oxapentyl- 1,4-en darstellen.

Beispiele für $R_3$ als Alkyl, das linear oder verzweigt sein kann, sind Methyl, Ethyl, n- und i-Propyl, n- und i-Butyl, Pentyl, Hexyl, Heptyl, Octyl, Nonyl, Decyl, Undecyl und Dodecyl.

Beispiele für $R_3$ als Alkenyl, das linear oder verzweigt sein kann, sind Vinyl, Crotonyl, Allyl, But-1-en-1-yl, But-1-en-2-yl, But-1-en-3-yl, But-1-en-4-yl, But-2-en-1-yl, But-2-en-2-yl, But-2-en-4-yl, Pentenyl, Hexenyl, Heptenyl, Octenyl, Nonenyl, Decenyl, Undecenyl und Dodecenyl.

Beispiele für $R_3$ als Alkinyl, das linear oder verzweigt sein kann, sind Ethinyl, Prop-2-in-1-yl, Prop-2-in-3-yl, Butinyl, Pentinyl, Hexinyl, Heptinyl, Octinyl, Decinyl und Dodecinyl.

$R_3$ enthält als Cycloalkyl und Cycloalkenyl bevorzugt 4-8 C-Atome, besonders 5 oder 6 C-Atome. Beispiele sind Cyclopropyl und Cyclopropenyl, Cyclobutyl und Cyclobutenyl, Cyclopentyl und Cyclopentenyl, Cyclohexyl und Cyclohexenyl, Cycloheptyl und Cycloheptenyl, Cyclooctyl und Cyclooctenyl.

$R_3$ enthält als Aryl bevorzugt 6 bis 12 C-Atome. Einige Beispiele sind Phenyl, Biphenyl und Naphthyl.

$R_3$ enthält als Heterocycloalkyl und Heterocycloalkenyl bevorzugt 4 bis 8, besonders 4 bis 6 Ring-C-Atome. Bevorzugte Heteroatome sind solche aus der Gruppe O, S und $NR_6$, worin $R_5$ H, $C_1$-$C_6$-Alkyl oder $C_1$-$C_7$-Acyl bedeutet. $R_3$ enthält als Heteroaryl bevorzugt 4 bis 11 Ring-C-Atome und bevorzugt Heteroatome aus der Gruppe O, S und -N=. Einige Beispiele für Heterocyclen sind Pyrrolidin, Tetrahydrofuran, Tetrahydrothiophen, Pyrrolin, Dihydrofuran, Dihydrothiophen, Indan, Dihydrocumaron, Dihydrobenzothiophen, Carbazol, Dibenzofuran, Dibenzothiophen, Pyrazolidin, Imidazolidin, Pyrazolin, Imidazolin, Benzimidazolidin, Oxazolidi, Oxazolin, Thiazolidin, Thiazolin, Isooxazolidin, Isooxazolin, Isothiazolidin, Isothiazolin, Benzoxazolidin, Benzisooxazolidi, Benzthiazolidin, 1,2,3- oder 1,2,4-Triazolidin, 1,2,3- oder 1,2,4-Triazolin, 1,2,3- oder 1,2,4-Oxazolidin oder -Oxazolin, Piperidin, Di- und Tetrahydropyridin, Dihydro- und Tetrahydropyran, Di- und Tetrahydrothiopyran, Piperazin, Dehydropiperazin, Morpholin, Thiomorpholin, 1,3- und 1,4-Dioxan, 1,4-Dithian, Azepan, 1,3-Dioxolan, 1,3-Dithiolan, Pyrrol, Indol, Imidazol, Benzimidazol, Furan, Thiophen, Benzofuran, Benzothiophen, Carbazol, Dibenzofuran, Dibenzothiophen, Oxazol, Isooxazol, Thiazol, Isothiazol, Benzoxazol, Benzothiazol, Pyridin, Pyrimidin, Pyrazin, Pyridazin, Chinolin, Isochinolin, Acridin, Chromen, Chroman, Pyran, Thiapyran, Phenazin, Phenoxazin, Phenolthiazin, Purin.

Beispiele für X in Formel II sind Methylen, Ethylen, 1,2- oder 1,3-Propylen, 1,2-, 1,3-oder 1,4-Butylen, Ethyliden, 1,1 - oder 2,2-Propyliden, 1,1- oder 2,2-Butyliden, Ethenylen, Prop-1-en-1,3- oder -1,2- oder -2,3-ylen.

Eine bevorzugte Ausführungsforme ist dadurch gekennzeichnet, dass es sich bei dem Salz um $Li^{\oplus}$- oder $NH_4^{\oplus}$-Salze von Carbonsäuren aus der Gruppe Furan-2-carbonsäure, Benzoesäure, Methylbenzoesäure, Phenylessigsäure, Zimtsäure, Sorbinsäure oder $C_2$-$C_8$-Alkancarbonsäuren handelt.

Die nicht salzartige organische Verbindung enthält bevorzugt 2 bis 20, besonders 2 bis 16 und insbesondere 2 bis 12 C-Atome. Insbesondere ist die organische Verbindung ein Ester einer organischen Monocarbonsäure mit insgesamt 2 bis 16 C-Atomen, ein Ether mit 2 bis 12 C-Atomen, ein Keton mit 3 bis 16 C-Atomen oder ein Alkohol mit 5 bis 16 C-Atomen.

Eine bevorzugte Ausführungsform ist dadurch gekennzeichnet, dass die organische Verbindung ein $C_1$-$C_6$-Alkylester von Furan-2-carbonsäure, Benzoesäure, Methylbenzoesäure, Phenylessigsäure, Zimtsäure,

Sorbinsäure oder $C_2$-$C_8$-Alkancarbonsäuren; ein $C_5$-$C_{12}$-Alkanol oder Benzylalkohol; ein dialiphatisches Keton mit 3 bis 10 C-Atomen, ein $C_1$ -$C_6$-Alkyl-phenylketon oder Diphenylketon; oder ein dialiphatischer Ether mit 2 bis 8 C-Atomen, $C_1$-$C_6$-Alkyl-phenylether oder Diphenylether ist.

Die Membran kann zur Durchführung des erfindungsgemässen Verfahrens in verschiedenen Formen ausgebildet und in Trennmodulen üblicher Bauart eingebaut sein. So kann man z.B. Flachmembranen oder asymmetrische Membranen zu Zwei- oder Mehrkammersystemen kombinieren. Man kann auch schlauchförmige Membranen oder Hohlfasern verwenden, die im allgemeinen in Form von Bündeln eingesetzt werden. Zur Erhöhung der mechanischen Stabilität können die Membranen auf ein Stützgerüst aufgebracht werden.

Das erfindungsgemässe Verfahren wird im allgemeinen bei Raumtemperatur durchgeführt. Zur Erzielung ausreichender Fliessgeschwindigkeit ist es vorteilhaft, auf der Seite der Lösung einen erhöhten Druck einzustellen. Der Druck beträgt bevorzugt 1 bis 10 MPa, besonders bevorzugt 1 bis 6 MPa. In einer besonderen Ausführungsform wird das Verfahren nach dem Gegenstromprinzip durchgeführt.

Das erfindungsgemässe Verfahren kann zum Beispiel als Anreicherungs- oder Reinigungsverfahren oder zur Rückgewinnung bzw. Abtrennung von Reaktionskomponenten oder Nebenprodukten aus Reaktionsrückständen oder Reaktionsgemischen eingesetzt werden oder zur Isolierung bzw. Reinigung von Zwischenprodukten verwendet werden, besonders wenn es sich um thermisch labile Substanzen handelt.

Die nachfolgenden Beispiele erläutern die Erfindung näher.

Beispiele 1-37:

a) Herstellung der Membranen

Als Ausgangsmaterial wird eine kommerziell erhältliche polymere Perfluorsulfonsäure-Membran (H-Form) verwendet (Nafion®-117, DuPont), die etwa 200 µm dick ist. Die Membran wird zur Ueberführung in die Salzform während 1 bis 30 Tagen jeweils in 1:1 Mischungen aus Methanol und einer einmolaren wässrigen Lösung des Hydroxids oder Chlorids des gewünschten Kations gelegt. Danach wird die Membran mit destilliertem Wasser und Methanol gewaschen. Vor der Verwendung wird die Membran bis zur Gleichgewichtsquellung ( 1 bis 5 Tage) in das jeweilige Lösungsmittel gelegt. Zur Herstellung von Membranen mit Silberkationen geht man von Na-Salzen der Perfluorsulfonsäure-Membran aus, die man mit Silbernitrat reagieren lässt.

b) Permeationsversuche

In der Mitte einer Druckzelle wird die Membran (Durchmesser 4,7 cm) in der Halterung befestigt. Jede Kammer ist mit einem Reservoir für die Lösung bzw. das reine Lösungsmittel verbunden, an die eine Pumpe angeschlossen ist. Die Reservoirs sind auf Waagen befestigt. Zwischen Reservoirs und Pumpen befinden sich Leitfähigkeitszellen und zwischen Auslauf und Reservoirs befinden sich UV-Detektoren. Auf der Seite mit dem Lösungsmittel wird ein Druck von 2,5 MPa eingestellt und die Lösung sowie das Lösungsmittel in gleicher Richtung zirkuliert. $^{14}C$-Messungen werden mit einem Flüssigscintillationsdetektor durchgeführt. Die Auswertung der Messdaten erfolgt mittels eines Computerprogramms. Die Detektoren werden jeweils mit der organischen Verbindung und dem Salz der Carbonsäure geeicht und die Eichkurven in Algorithmen umgewandelt, aus denen die gewünschten Daten errechnet werden.

Die Selektivität S ist wie folgt definiert, wobei $G^P$ Gewichtsanteil im Permeat und $G^L$ Gewichtsanteil in der Lösung bedeutet:

$$S = \frac{G^P \text{ (unpolarere organische Verbindung)}/G^P \text{ (Salz der Carbonsäure)}}{G^L \text{ (unpolarere organische Verbindung)}/G^L \text{ (Salz der Carbonsäure)}}$$

Weitere Angaben sind in den nachfolgenden Tabellen 1 und 2 enthalten.

Tabelle 1:

| Beispiel Nr. | Metallkation in der Membran | Lösungsmittel | Salz der Carbonsäure | Unpolarere organische Verbindung |
|---|---|---|---|---|
| 1 | $Li^{\oplus}$ | Methanol | Li-cinnamat | Methyl-cinnamat |
| 2 | $Li^{\oplus}$ | Methanol | Li-cinnamat | Methyl-cinnamat |
| 3 | $Li^{\oplus}$ | Methanol | Li-cinnamat | Methyl-cinnamat |
| 4 | $Li^{\oplus}$ | Methanol | Li-cinnamat | Methyl-cinnamat |
| 5 | $Li^{\oplus}$ | Methanol | Li-cinnamat | Methyl-cinnamat |
| 6 | $Li^{\oplus}$ | Methanol | Li-cinnamat | Methyl-cinnamat |
| 7 | $Li^{\oplus}$ | Methanol | Li-cinnamat | Methyl-cinnamat |
| 8 | $Li^{\oplus}$ | Methanol | Li-cinnamat | Methyl-cinnamat |
| 9 | $Li^{\oplus}$ | Methanol | Li-cinnamat | Methyl-cinnamat |
| 10 | $Li^{\oplus}$ | Methanol | Li-cinnamat | Furan-2-carbonsäure-ethylester |
| 11 | $Li^{\oplus}$ | Methanol | Li-cinnamat | Benzylalkohol |
| 12 | $Li^{\oplus}$ | Methanol | p-Toluyl-säure-Li-Salz | p-Toluylsäure-ethylester |
| 13 | $Li^{\oplus}$ | Methanol | Phenylessigsäure-Li-Salz | Phenylessigsäure-methylester |
| 14 | $Li^{\oplus}$ | Methanol | Benzoesäure-Li-Salz | Benzoesäure-methylester |
| 15 | $Li^{\oplus}$ | Methanol | Sorbinsäure-Li-Salz | Sorbinsäure-ethylester |
| 16 | $Li^{\oplus}$ | Methanol | Li-cinnamat | Acetophenon |
| 17 | $Li^{\oplus}$ | Methanol | Li-cinnamat | Methyl-phenyl-ether |
| 18 | $Li^{\oplus}$ | Methanol | Li-acetat | Methyl-acetat |
| 19 | $Li^{\oplus}$ | 2-Methoxy-ethanol | Li-cinnamat | Methyl-cinnamat |
| 20 | $Na^{\oplus}$ | Methanol | Li-cinnamat | Methyl-cinnamat |

Tabelle 1 (Fortsetzung)

| Beispiel Nr. | Metallkation in der Membran | Lösungsmittel | Salz der Carbonsäure | Unpolarere organische Verbindung |
|---|---|---|---|---|
| 21 | $Na^{\oplus}$ | Ethanol | Li-cinnamat | Methyl-cinnamat |
| 22 | $K^{\oplus}$ | Methanol | Li-cinnamat | Methyl-cinnamat |
| 23 | $K^{\oplus}$ | Methanol | Li-cinnamat | Methyl-acetat |
| 24 | $Ag^{\oplus}$ | Methanol | Li-cinnamat | Methyl-cinnamat |
| 25 | $Ag^{\oplus}$ | Methanol | Li-cinnamat | Methyl-cinnamat |
| 26 | $Cs^{\oplus}$ | Methanol | Li-cinnamat | Methyl-cinnamat |
| 27 | $Cs^{\oplus}$ | Methanol | Li-cinnamat | Methyl-cinnamat |
| 28 | $Ni^{2\oplus}$ | Methanol | Li-cinnamat | Methyl-cinnamat |
| 29 | $Co^{2\oplus}$ | Methanol | Li-cinnamat | Methyl-cinnamat |
| 30 | $Cu^{2\oplus}$ | Methanol | Li-cinnamat | Methyl-cinnamat |
| 31 | $NH_4^{\oplus}$ | Methanol | Li-cinnamat | Methyl-cinnamat |
| 32 | $N(C_4H_9)_4^{\oplus}$ | Methanol | Li-cinnamat | Methyl-cinnamat |
| 33 | Chinuclidinium (1-Azabicyclo [2.2.2] octan) | Methanol | Li-cinnamat | Methyl-cinnamat |
| 34 | $Li^{\oplus}$ | Methanol/Diethylenglycol dimethylether 1:1 | Ammonium-benzoat | Methyl-acetat |
| 35 | $Li^{\oplus}$ | Methanol | Li-benzoat | Acetophenon |
| 36 | $Li^{\oplus}$ | Methanol | Ammonium-benzoat | Methyl-benzoat |
| 37 | $Li^{\oplus}$ | Methanol | Li-cinnamat | Methyl-cinnamat |

Tabelle 1 (Fortsetzung)

| Beispiel Nr. | Metallkation in der Membran | Lösungsmittel | Salz der Carbonsäure | Unpolarere organische Verbindung |
|---|---|---|---|---|
| 38 | $Li^{\oplus}$ | Methanol | Octylammonium-cinnamat | Methyl-cinnamat |
| 39 | Octylammonium$^{\oplus}$ | Methanol | Li-cinnamat | Methyl-cinnamat |
| 40 | $Li^{\oplus}$ | Methanol | Di-Li-phthalat 1) | Methyl-benzoat |
| 41 | $Li^{\oplus}$ | Methanol | Di-Li-phthalat 1) | Tris(2-ethyl-hexyl)trimellitat |
| 42 | $Li^{\oplus}$ | Methanol | Tri-Li-trimellitat 2) | Dimethylphthalat |
| 43 | $Li^{\oplus}$ | Methanol | Tetra-Li-pyromellitat 3) | Methyl-benzoat |
| 44 | $Li^{\oplus}$ | Methanol | Di-Li-2-carboxy-cinnamat 4) | Methyl-cinnamat |

1) 98 % Di-Li-Salz + 2 % Mono-Li-Salz

2) 35 % Tri-Li-Salz + 65 % Di-Li-Salz

3) 45 % Tetra-Li-Salz + 55 % Tri-Li-Salz

4) 65 % Di-Li-Salz + 35 % Mono-Li-Salz

Tabelle 2:

| Beispiel Nr. | Permeationsgeschwindigkeit (mg• min$^{-1}$ •cm$^{-2}$) | Detektion [UV (nm)/ C 14] | Salz der Carbonsäuren (Menge Versuchsbeginn) | | Unpolarere organische Verbindung (Menge Versuchsbeginn) | | Versuchszeit (min.) | Versuchs-Ende: Mengen der Komponenten im Permeat (mg) | | Selektivität |
|---|---|---|---|---|---|---|---|---|---|---|
| | | | Menge (mg) | Konz. (Gew.%) | Menge (mg) | Konz. (Gew.%) | | Salz | organische Verbindung | |
| 1 | 1,675 | 305 | 48,75 | 0,00975 | 1,25 | 0,00025 | 1080 | 0,714 | 0,340 | 18,7 |
| 2 | 1,583 | 305 | 45,00 | 0,00900 | 5,00 | 0,00100 | 1080 | 0,280 | 0,342 | 11,0 |
| 3 | 1,737 | 305 | 35,00 | 0,00700 | 15,00 | 0,00300 | 1050 | 0,425 | 1,005 | 5,5 |
| 4 | 1,721 | 305 | 25,00 | 0,00500 | 25,00 | 0,00500 | 1080 | 0,399 | 1,321 | 3,3 |
| 5 | 1,725 | 305 | 15,00 | 0,00300 | 35,00 | 0,00700 | 1080 | 0,300 | 1,864 | 2,7 |
| 6 | 1,741 | 305 | 5,00 | 0,00100 | 45,00 | 0,00900 | 1080 | 0,159 | 2,484 | 1,7 |
| 7 | 1,749 | 305 | 1,25 | 0,00025 | 48,75 | 0,00975 | 1050 | 0,044 | 2,828 | 1,6 |
| 8 | 1,767 | 305 | 5,00 | 0,00100 | 5,00 | 0,00100 | 1080 | 0,085 | 0,276 | 3,3 |
| 9 | 1,732 | 305 | 250,00 | 0,05000 | 250,00 | 0,05000 | 1080 | 4,176 | 12,974 | 3,1 |
| 10 | 1,232 | 260 | 250,00 | 0,05000 | 250,00 | 0,05000 | 1080 | 2,379 | 14,467 | 6,1 |
| 11 | 1,207 | 260 | 250,00 | 0,05000 | 250,00 | 0,05000 | 1080 | 2,173 | 34,200 | 15,7 |
| 12 | 1,685 | 255 | 25,00 | 0,00500 | 25,00 | 0,00500 | 1080 | 0,495 | 1,473 | 3,0 |
| 13 | 1,653 | 250 | 250,00 | 0,05000 | 250,00 | 0,05000 | 1080 | 4,617 | 18,687 | 4,1 |
| 14 | 1,690 | 255 | 250,00 | 0,05000 | 250,00 | 0,05000 | 1080 | 4,465 | 19,138 | 4,3 |
| 15 | 1,680 | 275 | 75,00 | 0,00500 | 75,00 | 0,00500 | 1050 | 0,787 | 1,506 | 1,9 |
| 16 | 0,717 | 280 | 250,00 | 0,05000 | 250,00 | 0,05000 | 1080 | 2,165 | 17,186 | 7,9 |
| 17 | 1,083 | 280 | 250,00 | 0,00500 | 250,00 | 0,05000 | 1040 | 2,187 | 14,878 | 6,8 |
| 18 | 1,284 | C 14 | 7,50 | 0,00500 | 7,50 | 0,00500 | 1320 | 0,260 | 1,980 | 10,0 |
| 19 | 0,350 | 310 | 125,00 | 0,02500 | 125,00 | 0,02500 | 270 | 0,194 | 0,381 | 2,0 |
| 20 | 0,563 | 305 | 25,30 | 0,00500 | 25,30 | 0,00500 | 1080 | 0,170 | 0,569 | 3,4 |

Tabelle 2 (Fortsetzung)

| Beispiel Nr. | Permeationsgeschwindigkeit (mg• min$^{-1}$ •cm$^{-2}$) | Detektion [UV (nm)/ C 14] | Salz der Carbonsäuren (Menge Versuchsbeginn) Menge (mg) | Salz der Carbonsäuren Konz. (Gew.%) | Unpolarere organische Verbindung (Menge Versuchsbeginn) Menge (mg) | Unpolarere organische Verbindung Konz. (Gew.%) | Versuchszeit (min.) | Versuchs-Ende: Mengen der Komponenten im Permeat (mg) Salz | Versuchs-Ende: organische Verbindung | Selektivität |
|---|---|---|---|---|---|---|---|---|---|---|
| 21 | 0,067 | 300 | 25,00 | 0,00500 | 25,00 | 0,00500 | 1080 | 0,010 | 0,171 | 18,0 |
| 22 | 0,024 | 305 | 25,30 | 0,00500 | 25,30 | 0,00500 | 3240 | 0,295 | 0,443 | 1,5 |
| 23 | 0,010 | C 14 | 10,00 | 0,00500 | 10,00 | 0,00500 | 5730 | 0,049 | 0,302 | 6,5 |
| 24 | 0,358 | 305 | 25,30 | 0,00500 | 25,30 | 0,00500 | 6500 | 0,000 | 7,400 | ∞ |
| 25 | 0,189 | 305 | 1500,00 | 1,00000 | 3000,00 | 2,00000 | 1080 | 0,146 | 37,044 | 253,7 |
| 26 | 0,020 | 305 | 25,30 | 0,00500 | 25,30 | 0,00500 | 3600 | 0,390 | 0,000 | 0,0 |
| 27 | 0,037 | 305 | 1500,00 | 1,00000 | 1500,00 | 1,00000 | 260<br>340 | 0,234<br>0,260 | 0,000<br>0,077 | 0,0<br>0,3 |
| 28 | 0,031 | 305 | 25,30 | 0,00500 | 25,30 | 0,00500 | 2400 | 0,327 | 0,071 | 0,2 |
| 29 | 0,033 | 305 | 25,30 | 0,00500 | 25,30 | 0,00500 | 3510 | 0,772 | 0,466 | 0,6 |
| 30 | 0,064 | 305 | 25,30 | 0,00500 | 25,30 | 0,00500 | 2700 | 0,042 | 1,842 | 0,3 |
| 31 | 0,604 | 305 | 25,00 | 0,00500 | 25,00 | 0,00500 | 1080 | 0,331 | 0,719 | 2,2 |
| 32 | 0,314 | 305 | 25,00 | 0,00500 | 25,00 | 0,00500 | 1080 | 0,032 | 0,512 | 16,1 |
| 33 | 0,090 | 305 | 25,00 | 0,00500 | 25,00 | 0,00500 | 1080 | 0,040 | 0,116 | 2,9 |
| 34 | 0,352 | C 14 | 10,00 | 0,00500 | 10,00 | 0,00500 | 4350 | 1,880 | 3,780 | 2,6 |
| 35 | 0,945 | 255 | 250,00 | 0,05000 | 250,00 | 0,05000 | 1350 | 1,586 | 15,854 | 10,0 |
| 36 | 1,740 | 255 | 250,00 | 0,05000 | 250,00 | 0,05000 | 1680 | 8,839 | 29,195 | 3,3 |
| 37 | 1,422 | 305 | 5000,00 | 1.0000 | 5000,00 | 1,00000 | 60 | 4,800 | 7,672 | 1,6 |

Tabelle 2 (Fortsetzung)

| Beispiel Nr. | Permeationsgeschwindigkeit ($mg \cdot min^{-1} \cdot cm^{-2}$) | Detektion [UV (nm)/ C 14] | Salz der Carbonsäuren (Menge Versuchsbeginn) | | Unpolarere organische Verbindung (Menge Versuchsbeginn) | | Versuchszeit | Versuchs-Ende: Mengen der Komponenten im Permeat (mg) | | Selektivität |
|---|---|---|---|---|---|---|---|---|---|---|
| | | | Menge (mg) | Konz. (Gew.%) | Menge (mg) | Konz. (Gew.%) | (min.) | Salz | organische Verbindung | |
| 38 | 1,483 | 305 | 25,00 | 0,00500 | 25,00 | 0,00500 | 2070 | 1,024 | 3,093 | 3,3 |
| 39 | 1,099 | 305 | 25,00 | 0,00500 | 25,00 | 0,00500 | 2040 | 0,441 | 2,602 | 6,5 |
| 40 | 1,400 | 270 | 75,00 | 0,01500 | 75,00 | 0,01500 | 2040 | 1,216 | 14,340 | 14,3 |
| 41 | 1,392 | 270 | 75,00 | 0,01500 | 75,00 | 0,01500 | 2040 | 1,547 | 10,779 | 8,0 |
| 42 | 1,464 | 270 | 75,00 | 0,01500 | 75,00 | 0,01500 | 2040 | 1,601 | 13,096 | 9,7 |
| 43 | 1,587 | 270 | 2,50 | 0,00050 | 2,50 | 0,00050 | 2520 | 0,001 | 0,105 | 109,6 |
| 44 | 1,563 | 305 | 75,00 | 0,01500 | 75,00 | 0,01500 | 2040 | 1,434 | 9,361 | 7,3 |

## Patentansprüche

1. Verfahren zum Anreichern oder Trennen von Salzen organischer Carbonsäuren von nicht salzartigen organischen Verbindungen mit einer semipermeablen Membran aus einem Perfluorsulfonsäurepolymer oder Salzen dieser Polymeren, dadurch gekennzeichnet, dass man eine Lösung der Salze und organischen Verbindungen in einem unsubstituierten oder mit $C_1$-$C_3$-Alkoxy substituierten $C_1$-$C_4$-Alkanol, Mischungen dieser Alkanole oder Mischungen der Alkanole mit Ethern mit einer Seite der Membran in Berührung bringt und sich auf der Gegenseite der Membran das reine Lösungsmittel befindet.

2. Verfahren gemäss Anspruch 1, dadurch gekennzeichnet, dass die Konzentration der Salze und organischen Verbindungen 0,0001 bis 10 Gewichtsprozent beträgt, bezogen auf die Lösung.

3. Verfahren gemäss Anspruch 1, dadurch gekennzeichnet, dass die Membran eine Dicke von 5 bis 300 μm aufweist.

4. Verfahren gemäss Anspruch 1, dadurch gekennzeichnet, dass die Membran aus einem Perfluorsulfonsäurepolymer mit wiederkehrenden Strukturelementen der Formel I

$$-\!\!\left[CF_2\text{-}C(R_1)_2\right]_w\!\!-CF_2\text{-}CF\text{-} \quad \left\lfloor\, \left(O\text{-}CF_2\text{-}CFR_2\right)_x\left[O(CFR_2)_y\right]_z\!-SO_3M \right. \qquad (I)$$

besteht, worin die $R_1$ und $R_2$ unabhängig voneinander für F oder $C_1$-$C_{10}$-Perfluoralkyl stehen, w eine Zahl von 5 bis 15 bedeutet, x eine Zahl von 0 bis 6 darstellt, y eine Zahl von 1 bis 16 darstellt, z eine Zahl von 0 bis 16 ist, und M für $H^{\oplus}$, ein Ammoniumkation oder ein Metallkation steht.

5. Verfahren gemäss Anspruch 4, dadurch gekennzeichnet, dass M für $NH_4^{\oplus}$, ein Ammoniumkation mit insgesamt 1 bis 18 C-Atomen, oder ein ein- bis dreiwertiges Metallkation steht.

6. Verfahren gemäss Anspruch 4, dadurch gekennzeichnet, dass M für ein Alkalimetallkation oder $Ag^{\oplus}$ steht.

7. Verfahren gemäss Anspruch 1, dadurch gekennzeichnet, dass das Lösungsmittel Methanol, Ethanol, 1- oder 2-Propanol oder 2-Methoxyethanol ist.

8. Verfahren gemäss Anspruch 1, dadurch gekennzeichnet, dass es sich bei den Salzen der organischen Carbonsäure um Ammonium- oder Metallsalze handelt.

9. Verfahren gemäss Anspruch 8, dadurch gekennzeichnet, dass das Salz ein Alkalimetallsalz oder ein Ammoniumsalz ist.

10. Verfahren gemäss Anspruch 8, dadurch gekennzeichnet, dass es sich um ein $NH_4^{\oplus}$- Salz oder $Li^{\oplus}$-Salz handelt.

11. Verfahren gemäss Anspruch 1, dadurch gekennzeichnet, dass es sich bei der organischen Carbonsäure um eine aliphatische, cycloaliphatische, aromatische, heterocyclische oder heteroaromatische Monocarbonsäure mit 1 bis 18 C-Atomen handelt.

12. Verfahren gemäss Anspruch 11, dadurch gekennzeichnet, dass die organische Säure der Formel (II)

$$R_3\text{-X-COOH} \qquad \text{(II)}$$

entspricht, worin X eine direkte Bindung, $C_1$-$C_4$-Alkylen, $C_2$-$C_4$-Alkyliden oder $C_2$-$C_4$-Alkenylen bedeutet, $R_3$ für H, $C_1$-$C_{12}$-Alkyl, $C_2$-$C_{12}$-Alkenyl, $C_2$-$C_{12}$-Alkinyl, $C_3$-$C_{12}$-Cycloalkyl, $C_3$-$C_{12}$-Cycloalkenyl, $C_6$-$C_{16}$-Aryl, $C_3$-$C_{12}$-Heterocycloalkyl, $C_3$-$C_{12}$-Heterocycloalkenyl, $C_6$-$C_{16}$-Heteroaryl steht, die unsubstituiert oder mit -OH, -SH, -CN, $-NO_2$, Halogen, $C_1$-$C_6$-Alkyl, $C_1$-$C_6$-Alkoxy, $C_1$-$C_6$-Alkylthio, $C_1$-$C_6$-Alkyl-Y- mit Y gleich -CO-, -SO-, $-SO_2$-, -CO-O-, -O-CO-, $-CO-NR_4R_5$-, $-NR_4R_5-CO$- substituiert sind, und $R_4$ und $R_5$ unabhängig voneinander H, $C_1$-$C_6$-Alkyl, $C_2$-$C_4$-Hydroxyalkyl oder $R_4$ und $R_5$ zusammen Tetramethylen, Pentamethylen oder 3-Oxapentyl-1,4-en darstellen.

13. Verfahren gemäss Anspruch 1, dadurch gekennzeichnet, dass es sich bei dem Salz um $Li^{\oplus}$- oder $NH_4^{\oplus}$-Salze von Carbonsäuren aus der Gruppe Furan-2-carbonsäure, Benzoesäure, Methylbenzoesäure, Phenylessigsäure, Zimtsäure, Sorbinsäure oder $C_2$-$C_8$-Alkancarbonsäuren handelt.

14. Verfahren gemäss Anspruch 1, dadurch gekennzeichnet, dass die nicht salzartige organische Verbindung ein Ester einer organischen Monocarbonsäure mit insgesamt 2 bis 16 C-Atomen, ein Ether mit 2 bis 12 C-Atomen, ein Keton mit 3 bis 16 C-Atomen oder ein Alkohol mit 5 bis 16 C-Atomen ist.

15. Verfahren gemäss Anspruch 14, dadurch gekennzeichnet, dass die nicht salzartige organische Verbindung ein $C_1$-$C_6$-Alkylester von Furan-2-carbonsäure, Benzoesäure, Methylbenzoesäure, Phenylessigsäure, Sorbinsäure oder $C_2$-$C_8$-Alkancarbonsäuren; ein $C_5$-$C_{12}$-Alkanol oder Benzylalkohol; ein dialiphatisches Keton mit 3 bis 10 C-Atomen, ein $C_1$-$C_6$-Alkyl-phenylketon oder Diphenylketon; oder ein dialiphatischer Ether mit 2 bis 8 C-Atomen, $C_1$-$C_6$-Alkyl-phenylether oder Diphenylether ist.

16. Verfahren gemäss Anspruch 1, dadurch gekennzeichnet, dass es bei Raumtemperatur durchgeführt wird.

17. Verfahren gemäss Anspruch 1, dadurch gekennzeichnet, dass auf der Seite mit der Lösung ein Druck von 1 MPa bis 10 MPa eingestellt wird.

18. Verfahren gemäss Anspruch 1, dadurch gekennzeichnet, dass es im Gegenstrom durchgeführt wird.